# EUROPÄISCHE PATENTSCHRIFT

(11) **EP 0 629 584 B1**
(45) Veröffentlichungstag und Bekanntmachung des Hinweises auf die Patenterteilung: **16.07.1997**
(21) Anmeldenummer: 94109128.2
(22) Anmeldetag: 14.06.1994
(51) Int. Cl.: C02F 3/28

(54) **Biogasreaktor zur anaeroben Behandlung von Abwässern**
Biogas reactor for anaerobic waste water treatment
Réacteur produisant du biogaz pour le traitement anaérobie des eaux usées

(30) Priorität: 17.06.1993 DE 4320096
(43) Veröffentlichungstag der Anmeldung: 21.12.1994
(73) Patentinhaber: PASSAVANT-WERKE AG, D-65322 Aarbergen 7 (DE)
(72) Erfinder: Coenon, Hubert, Dr. Ing., D-65326 Aarbergon 2 (DE); Hubert, Helmut, Dr.-Ing., D-65197 Wiesbaden (DE); Zumbrägel, Michael, Dipl.-Ing., D-65326 Aarborgen 2 (DE)

(56) Entgegenhaltungen:
- EP-A- 0 244 029
- DE-A- 2 728 585
- FR-A- 2 416 200

## Beschreibung

Die Erfindung betrifft Biogasreaktoren für das unter der Bezeichnung "UPFLOW" bekanntgewordene Verfahren zur anaeroben Behandlung von Abwässern. Die im Aufstrom betriebenen Biogasreaktoren besitzen im oberen Teil mindestens eine Auffanghaube für das Biogas, in der im Betrieb durch Gasgegendruck ein Flüssigkeitsspiegel aufgebaut und aufrechterhalten wird. Die Auffanghauben haben einen vertikalen Querschnitt eines umgekehrten Trichters (DE-A 27 28 585). Auch halbrohrförmige Querschnitte sind bekannt.

Die bekannten Querschnitte der Auffanghauben haben den Nachteil, daß sich die Größe der Trennfläche zwischen Gas und Flüssigkeit beim Schwanken des Flüssigkeitsspiegels ständig ändert. Der Gasaustritt aus der Flüssigkeit ist bei hohem Flüssigkeitsspiegel wegen der geringen Fläche stark behindert. Im Extremfall kann es infolge hoher Turbulenzen zum Übertritt von Biomasse in den Gasraum kommen. Ein anderer unerwünschter Effekt ist die Neigung zur Schaumbildung. Die Aufgabe, diese Nachteile zu beseitigen, wird erfindungsgemäß dadurch gelöst, daß die Auffanghaube zumindest im Schwankungsbereich des Flüssigkeitsspiegels gleichbleibenden horizontalen Querschnitt hat. Zusätzlich kann die Oberseite der Auffanghaube mit das Abgleiten des Bioschlamms ermöglichenden Schrägflächen versehen sein. Die Phasentrennfläche für den Gasübergang bleibt also bei allen Höhenlagen des Flüssigkeitsspiegels gleich. Die auf die Oberseite der Auffanghauben als Dach wieder aufgesetzten Gleitflächen für den Schlamm können zur Aufnahme von Biege- und Querkräften herangezogen werden.

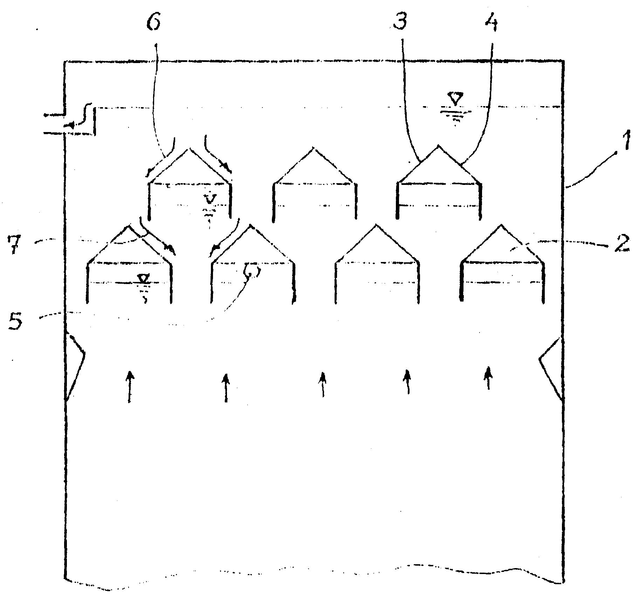

Die Abbildung zeigt einen Vertikalschnitt durch einen Biogasreaktor 1 mit erfindungsgemäß gestalteten Auffanghauben 2, die in zwei Ebenen, sich überlappend, gestaffelt sind. Die Auffanghauben haben einen nach unten offenen U-Querschnitt mit scharfen Ecken, auf den zwei Schrägflächen 3, 4 dachförmig aufgesetzt sind. Der Abzug 5, des aufgefangenen Biogases liegt vorzugsweise an der Stirnseite der Abzugshauben. Der in dem verlangsamt aufwärts strömenden Abwasser noch enthaltene Schlamm sedimentiert in den strömungsarmen Zonen über den Auffanghauben auf die schrägen Gleitflächen und rutscht entlang der Pfeile 6, 7 zurück ins Schlammbett.

## Patentansprüche

1. Biogasreaktor zur anaeroben Behandlung von Abwässern, enthaltend mindestens eine in das Abwasser eingetauchte Auffanghaube für beim biologischen Abbau der Abwasserinhaltsstoffe gebildetes Biogas, in der durch Gasgegendruck ein im Betrieb schwankender Flüssigkeltsspiegel aufgebaut und aufrechterhalten wird, **dadurch gekennzeichnet**, daß die Auffanghaube (2) zumindest in dem Schwankungsbereich des Flüssigkeitsspiegels gleichbleibenden horizontalen Querschnitt hat.

2. Biogasreaktor nach Anspruch 1, **dadurch gekennzeichnet**, daß die Oberseite der Auffanghauben (2) mit das Abgleiten von Bioschlamms ermöglichenden Schrägflächen (3, 4) versehen ist.

3. Biogasreaktor nach Anspruch 2, **dadurch gekennzeichnet**, daß die Schrägflächen (3, 4) dachförmig ausgebildet sind.

4. Biogasreaktor nach Anspruch 2 oder 3, **dadurch gekennzeichnet**, daß die Schrägflächen so ausgebildet sind, daß sie zur Aufnahme von Biege- und Querkräften geeignet sind.

## Claims

1. Biogas reactor for the anaerobic treatment of waste water, containing at least one collection hood dipping into the waste water for biogas formed during the biological decomposition of the waste water components, in which is built up and maintained a liquid that fluctuates during operation through gas counterpressure, characterized in that the collection hood (2) has, at least in the oscillation region of the liquid level, a horizontal cross-section that remains constant.

2. Biogas reactor according to Claim 1, characterized in that the topside of the collection hoods (2) is provided with sloping surfaces (3, 4) that allow the biosludge to slide off.

3. Biogas reactor according to Claim 2, characterized in that the sloping surfaces (3, 4) are shaped like a roof.

4. Biogas reactor according to Claims 2 or 3, characterized in that the sloping surfaces are so formed as to be suitable for absorbing flexion and transverse forces.

## Revendications

1. Réacteur à biogas destiné au traitement anaérobie des eaux usées comprenant au moins un capot collecteur immergé dans l'eau usée pour le biogas formé lors du traitement anaérobie des substances contenues dans l'eau usée, dans lequel se produit et est maintenu par la contre-pression du biogas un niveau d'eau variant lors du service, **caractérisé en ce que** la section horizontale du capot collecteur (2) est constant au moins dans l'amplitude de variation du niveau d'eau.

2. Réacteur à biogas selon revendication 1, **caractérisé en ce que** la face supérieure des capots collecteurs (2) est inclinée pour faciliter le glissement des boues biologiques.

3. Réacteur à biogas selon revendication 2, **caractérisé en ce que** les surfaces inclinées (3, 4) forment un toit.

4. Réacteur à biogas selon une des revendications 2 ou 3, **caractérisé en ce que** les surfaces inclinées sont formées de sorte qu'elles sont capables de recevoir des forces laterales et transversales.
